Europäisches Patentamt

European Patent Office

Office européen des brevets

(19)

(11) Veröffentlichungsnummer: **0 123 296**
**B1**

(12) # EUROPÄISCHE PATENTSCHRIFT

(45) Veröffentlichungstag der Patentschrift:
**17.02.88**

(51) Int. Cl.⁴: **A 61 K 7/16**

(21) Anmeldenummer: **84104473.8**

(22) Anmeldetag: **19.04.84**

(54) **Verwendung von Hesperidin.**

(30) Priorität: **25.04.83 DE 3314895**

(43) Veröffentlichungstag der Anmeldung:
**31.10.84 Patentblatt 84/44**

(45) Bekanntmachung des Hinweises auf die Patenterteilung:
**17.02.88 Patentblatt 88/7**

(84) Benannte Vertragsstaaten:
**AT BE CH DE FR GB IT LI LU NL SE**

(56) Entgegenhaltungen:
**DE - A - 2 523 866
DE - A - 3 241 017**

**CHEMICAL ABSTRACTS, Band 63, 1965, Spalte 7526d, Columbus, Ohio, USA; D.T. THOMPSON et al.: "Certain organic substances and their effects on the incidence of dental caries in the cotton rat"
CHEMICAL ABSTRACTS, Band 92, Nr. 24, Juni 1980, Seite 322, Nr. 203427s, Columbus, Ohio, USA**

(73) Patentinhaber: **Blendax-Werke R. Schneider GmbH & Co., Rheinallee 88, D-6500 Mainz (DE)**

(72) Erfinder: **Raaf, Helmut, Dr., Schönblick 7, D-6551 Bockenau (DE)**

**Beschreibung**

Die vorliegende Erfindung betrifft die Verwendung von Hesperidin zur Herstellung von Zahn- und Mundpflegemitteln, die auf das Zahnfleisch eine gesunderhaltende Wirkung ausüben, die Bildung von Zahnbelag hemmen und so insbesondere auch die Ursachen für Zahnfleischerkrankungen beseitigen.

Mund- und Zahnpflegemittel mit derartiger Zielsetzung sind bereits seit langem bekannt und auch im Handel.

Für diese Zahnpasten wurden verschiedene Wirkstoffe vorgeschlagen.

Aus der DE-A-2 523 866 sind bereits schweisshemmende Mittel bekannt, welche einen Gehalt an Hesperidin aufweisen. Andererseits sind aus C.A. 63, 7526d (1965) Tierversuche bekanntgeworden, mittels derer unter anderen Stoffen das Hesperidin auf seine Wirksamkeit zur Kariesprophylaxe untersucht wurde.

Es wurde nunmehr gefunden, dass durch einen Zusatz von Hesperidin, vorzugsweise in einer Menge zwischen 0,05 und 1,5 Gew.-%, vorzugsweise 0,1 bis 1,0, insbesondere 0,15 bis 0,5 Gew.-% der Gesamtzusammensetzung, des erfindungsgemässen Zahn- und Mundpflegemittels eine ausgezeichnete zahnfleischpflegende Wirkung, insbesondere im Hinblick auf die Bekämpfung bzw. Verhinderung von Zahnfleischbluten, erzielt werden kann.

Diese Wirkung lässt sich sogar noch weiter steigern im Sinne eines synergistischen Effekts, wenn Hesperidin in dem erfindungsgemässen Zahn- und Mundpflegemittel zusammen mit Harnstoff eingesetzt wird. Die Wirkung von Harnstoff alleine, die auch Nutzen in der Praxis gefunden hat, ist an sich bekannt. Von dieser Substanz ist nicht nur beschrieben, dass sie eine kariesprophylaktische und zahnsteinverhütende Wirkung aufweist (vgl. Journal of Periodontology, Vol. 37 (1966), S. 20–33), sondern sie besitzt auch eine keratinisierende Wirkung, d.h., beim Einsatz in Zahn- und Mundpflegemitteln in genügender Konzentration wirkt sie auf das Zahnfleisch festigend, was sich durch eine Erhöhung des Keratinisations-Indexes signifikant nachweisen lässt.

Es war jedoch überraschend, dass sich die an sich schon überraschende Wirksamkeit von Hesperidin noch synergistisch steigern lässt.

Eine besonders günstige Wirkung wurde erreicht, wenn eine Zahnpasta mit einem Gehalt von mindestens 5 bis 15 Gew.-% Harnstoff, vorzugsweise 8 Gew.-% mit etwa 0,1 bis etwa 1, vorzugsweise etwa 0,1 bis 0,5 Gew.-% Hesperidin, bezogen auf die Gesamtzusammensetzung, einer klinischen Untersuchung unterzogen wurde.

Hesperidin ist bekanntlich ein Flavanon der Struktur 3′,5,7-Trihydroxy-4′-methoxyflavanon-7-[6-(β-L-rhamnosido)-D-glucosid], das beispielsweise aus den Fruchtschalen unreifer Orangen gewonnen werden kann (vgl. Römpp's Chemie-Lexikon, 7. Auflage, S. 1458).

Unter der Bezeichnung Hesperidin werden nicht nur das Glykosid selbst, sondern auch seine pharmakologisch aktiven Derivate, insbesondere die Ester, beispielsweise der Mono- und Diphosphorsäureester, das Ascorbat und deren Salze, verstanden, wobei die angegebene Menge jedoch jeweils auf Hesperidin zu beziehen ist.

Wie bereits ausgeführt, liegt die bevorzugte Menge an Hesperidin in den erfindungsgemässen Zahn- und Mundpflegemitteln bei etwa 0,05 bis 1,5 Gew.-% der Gesamtzusammensetzung; eine optimale Dosierung liegt zwischen etwa 0,1 und 1 Gew.-%, beispielsweise bei 0,15 bis 0,5 Gew.-% der Gesamtzusammensetzung.

Der fakultative Anteil an Harnstoff in dem erfindungsgemäss herstellbaren Zahn- und Mundpflegemittel liegt vorzugsweise bei mindestens 5 Gew.-% der Gesamtzusammensetzung; eine besonders günstige Wirkung hat sich bei der Kombination von 8% Harnstoff mit 0,2 Gew.-% Hesperidin herausgestellt. Im allgemeinen wird der Gehalt an Harnstoff 15 Gew.-%, vorzugsweise 10 Gew.-%, bezogen auf die Gesamtzusammensetzung, nicht überschritten.

Obwohl prinzipiell jede geeignete Applikationsform eines Zahn- und Mundpflegemittels mit einem Gehalt an Harnstoff und Hesperidin verwendet werden kann, wie beispielsweise Mundspray oder Zahnpulver, wird den Applikationsformen Zahnpasta und Mundwasser der Vorzug gegeben.

Eine solche Zahnpasta kann opak oder eine durch Verwendung geeigneter, in ihren Brechungsindices mit dem Brechungsindex des Trägermaterials übereinstimmender Poliermittel hergestellte transparente Zahnpasta sein.

Eine besonders geeignete Zahnpasta ist in der LU-A-82 933 beschrieben; diese enthält das Poliermittel Calciumcarbonat sowie mindestens 5 Gew.-% Harnstoff, etwa 0,5 bis 1,6 Gew.-% eines Alkalisalzes einer höheren Fettsäure mit etwa 12 bis etwa 18 Kohlenstoffatomen, ist im wesentlichen frei von synthetischen Tensiden und weist einen pH-Wert im alkalischen Bereich von mindestens 7,5, vorzugsweise zwischen 7,5 und 9,5 auf.

Es ist jedoch auch möglich, Zahnpasten auf anderer Pastengrundlage einzusetzen, die als Poliermittel beispielsweise Alkalialuminiumsilikate wie solche vom Zeolith-Typ A, beschrieben in den EP-B-2690 und 3023, verschiedene Calciumphosphate wie Dicalciumorthophosphat in Form seines Dihydrats oder wasserfrei, Tricalciumphosphat, Calciumpyrophosphat, unlösliche Alkalimetaphosphate, Aluminiumoxid oder Aluminiumoxidtrihydrat, Siliciumdioxide verschiedener Modifikationen wie Siliciumdioxid-Xerogele, -Hydrogele oder gefällte Siliciumdioxide, oder pulverförmige Kunststoffe enthalten.

Es können selbstverständlich auch Poliermittelgemische aus den genannten Substanzen eingesetzt werden, beispielsweise ein Gemisch aus Calciumcarbonat und synthetischem Zeolith A im Verhältnis von etwa 1:1.

Der Poliermittelanteil in den erfindungsgemäss herstellbaren Zahnpasten liegt vorzugsweise zwischen etwa 20 und 60 Gew.-% der Gesamtzusammensetzung.

Wie bereits angedeutet, stellt es eine bevorzugte Ausführungsform der vorliegenden Erfindung dar, Hesperidin oder eine synergistische Kombination aus Harnstoff und Hesperidin solchen Zahnpasten einzuverleiben, die nur einen geringen oder keinen Anteil an synthetischen oberflächenaktiven Substanzen enthalten, sondern gegebenenfalls Alkalisalze höherer Fettsäuren, beispielsweise solche von Laurinsäure, Myristinsäure, Palmitinsäure, Stearinsäure oder Gemischen derselben, beispielsweise Cocosölfettsäuren oder Talgfettsäuren, enthalten. Derartige Salze höherer Fettsäuren sind vorzugsweise in einer Menge zwischen etwa 0,5 und 1,5 Gew.-% der Gesamtzusammensetzung vorhanden.

Es ist jedoch selbstverständlich auch möglich, die üblichen in Zahnpasten eingesetzten oberflächenaktiven Verbindungen in Mengen bis zu etwa 2 Gew.-% der Gesamtzusammensetzung zu verwenden, gegebenenfalls im Gemisch mit den genannten Fettsäuresalzen. Solche synthetischen oberflächenaktiven Stoffe sind beispielsweise Alkylsulfate, Aklylethersulfate, Olefinsulfonate, Natriumlauroylsarcosinat oder ampholytische, nichtionische oder kationaktive Verbindungen.

Eine Übersicht über in Zahnpasten einsetzbare Zusammensetzungen findet sich, wie überhaupt über sonstige zur Herstellung von Zahnpflegemitteln üblicherweise zum Einsatz gelangende Stoffe und die dabei angewandten Herstellungsverfahren, in dem Handbuch von M.S. Balsam und E. Sagarin «Cosmetics – Science and Technology», 2nd Ed., Vol. 1, S. 423 bis 533, (1972), auf das hier ausdrücklich Bezug genommen wird.

Gleiches gilt hinsichtlich der in Zahnpasten üblicherweise in Mengen zwischen etwa 10 und etwa 35 Gew.-% zum Einsatz gelangenden Feuchthaltemittel, wie Glycerin, Diole wie 1,4-Butandiol oder 1,2-Propandiol oder Zuckeralkohole wie Sorbit, Mannit oder Xylit und Polyglykole mit niederen Molekulargewichten, ebenso für Verdickungsmittel, deren Mengenanteil in Zahnpasten zwischen etwa 0,25 und etwa 5 Gew.-% der Gesamtzusammensetzung liegt.

Bevorzugte Verdickungsmittel sind Carboxymethylcellulose und deren Alkalisalze, insbesondere Natriumcarboxymethylcellulose, Hydroxyalkylcellulosen wie Hydroxymethylcellulose und Hydroxyethylcellulose, Methylcellulose, Pflanzengummen wie Tragant, Gummi arabicum, Carayagummi, Guargummi, Xanthangummi und Irish Moos, synthetische Polyelektrolyte wie die Alkalisalze der Polyacrylsäure sowie anorganische Verdickungsmittel, beispielsweise kolloidales Magnesiumaluminiumsilikat oder disperses Siliciumdioxid.

In den erfindungsgemässen Zahn- und Mundpflegemitteln können selbstverständlich auch weitere Wirkstoffe Verwendung finden. Solche sind insbesondere die bekannten kariesprophylaktischen Fluoride, vorzugsweise in einer solchen Menge, dass die Konzentration an reinem Fluor im Mittel etwa 0,05 bis etwa 1 Gew.-%, vorzugsweise 0,1 bis 0,5 Gew.-% des Mittels beträgt.

Geeignete Fluorverbindungen sind insbesondere die verschiedenen Salze der Monofluorphosphorsäure, insbesondere Natrium-, Kalium-, Lithium-, Calcium- und Aluminiummono- und difluorphosphat sowie die verschiedenen, Fluor in ionisch gebundener Form enthaltenen Fluoride, insbesonder Alkalifluoride wie Natrium-, Lithium-, Kalium- und Ammoniumfluorid, Zinnfluorid, Manganfluorid, Kupferfluorid, Zirkoniumfluorid und Aluminiumfluorid sowie Gemische oder Anlagerungsprodukte dieser Fluoride untereinander und mit anderen Fluorverbindungen, beispielsweise Alkalimanganfluoride.

Auch organische Fluorverbindungen können mit Erfolg eingesetzt werden, insbesondere die bekannten Additionsprodukte aus langkettigen Aminen oder Aminosäuren und Fluorwasserstoff, Monoethanolamindihydrofluorid oder Monoethyltriethylammoniumfluorid.

Weitere, in den erfindungsgemäss hergestellten Zahn- und Mundpflegemitteln einsetzbare Stoffe sind Zahnbelag entfernende Substanzen, beispielsweise die unter den Trivialnamen «Chlorhexidin» oder «Alexidin» bekannten Bisguanide 1,6-Di-4'-(chlorphenyldiguanido)hexan oder 1,6-Di-(2-ethylhexylidiguanido)hexan bzw. deren vorzugsweise wasserlöslichen Salze, Mittel zur Verhinderung von Zahnsteinbildung wie Hydroxyethan-1,1-diphosphonsäure oder Alkylentetramethylenphosphonsäuren und deren wasserlösliche Salze, Allantoin, Azulen, etc.

Im folgenden werden einige Beispiele gegeben, die das Wesen des Gegenstandes der vorliegenden Anmeldung und ihre vorteilhafte Wirkung charakterisieren:

Beispiel 1

| Opake Zahnpasta | (Gew.-%) |
|---|---|
| Methylhydroxyethylcellulose | 1,00 |
| Calciumcarbonat | 42,00 |
| Harnstoff | 8,00 |
| Allantoin | 0,30 |
| Natriumlaurat | 0,65 |
| Natriumbenzoat | 0,30 |
| Methyl-p-hydroxybenzoat | 0,15 |
| Saccharin-Natrium | 0,05 |
| Sorbit, 70%-ig | 8,00 |
| Hesperidin | 0,60 |
| Kolloidales Siliciumdioxid | 0,35 |
| Aromastoffe | 1,00 |
| Wasser | 37,60 |

Beispiel 2

| Opake Zahnpasta | (Gew.-%) |
|---|---|
| Harnstoff | 6,00 |
| Natriummonofluorphosphat | 0,75 |
| Allantoin | 0,10 |
| Natriumstearat/-laurat (1:1) | 0,70 |
| Natriumbenzoat | 0,25 |
| Calciumcarbonat | 40,00 |
| Methyl-p-hydroxybenzoat | 0,15 |
| Natriumcyclamat | 0,10 |
| Calciumsilikat | 0,50 |
| Hydroxyethylcellulose | 1,10 |
| Sorbit, 70%-ig | 9,00 |

| | |
|---|---|
| Aromagemisch | 1,00 |
| Hesperidin | 0,55 |
| Wasser | 39,80 |

**Beispiel 3**

| Opake Zahnpasta | (Gew.-%) |
|---|---|
| Calciumcarbonat | 22,50 |
| Synthetischer Zeolith A (entsprechend EP-PS 3023; $Na_{12}(AlO_2)_{12}(SiO_2)_{12} \cdot 27H_2O$) | 16,50 |
| Sorbit, 70%-ig | 17,50 |
| Glycerin | 3,50 |
| Carboxymethylcellulose | 1,20 |
| Benzoesäure | 0,30 |
| Methyl-p-hydroxybenzoat | 0,10 |
| Saccharin-Natrium | 0,05 |
| Natriummonofluorphosphat | 1,25 |
| Kolloidale Kieselsäure | 0,20 |
| Hesperidinascorbat | 0,80 |
| Aromastoffe | 1,00 |
| Natriumlaurylsulfat, 86%-ig | 1,20 |
| Wasser | 33,90 |

**Beispiel 4**

| Transparente Zahnpasta | (Gew.%) |
|---|---|
| Carboxymethylcellulose | 0,50 |
| Natriumbenzoat | 0,15 |
| Polyethylenglykol 400 | 5,00 |
| Glycerin | 45,00 |
| Harnstoff | 7,50 |
| Hesperidinmonophosphat, Natriumsalz | 0,95 |
| Allantoin | 0,15 |
| Guajazulen | 0,05 |
| Saccharin-Natrium | 0,07 |
| Natriumlauroylsarkosinat | 1,10 |
| Phenylsalicylat | 0,10 |
| Aromagemisch | 1,00 |
| 10%-ige blaue Farbstoff-Lösung | 0,03 |
| Dehydratisiertes Siliciumdioxid-Gel (Oberfläche 290m²/g; Mittl. Teilchendurchmesser 6 μ) | 20,00 |
| Wasser | 18,40 |

**Beispiel 5**

| Opake Zahnpasta | (Gew.-%) |
|---|---|
| Chlorhexidindigluconat | 0,10 |
| Hesperidin | 0,65 |
| Allantoin | 0,20 |
| Natriumfluorid | 0,30 |
| Aluminiumoxidtrihydrat | 25,00 |
| Zeolith A (nach EP-B-3023; $Na_{12}(AlO_2)_{12}(SiO_2)_{12} \cdot 27H_2O$) | 15,00 |
| Medizinische Seife (DAB 6) | 0,70 |
| Natriumsulforicinoleat | 0,30 |
| Hydroxyethylcellulose | 1,05 |
| N-Prophyl-p-hydroxybenzoat | 0,15 |
| Methyl-p-hydroxybenzoat | 0,15 |
| Natriumbenzoat | 0,15 |
| Saccharin-Natrium | 0,05 |
| Glycerin | 8,50 |
| Sorbit, 70%-ig | 7,50 |
| Aromazusammensetzung | 1,20 |
| Wasser | 39,00 |

**Beispiel 6**

| Mundwasserkonzentrat | (Gew.-%) |
|---|---|
| Diphosphorsäureester des Hesperidins, Natriumsalz | 1,60 |
| Aromagemisch | 3,50 |
| Nichtionogener Emulgator | 6,00 |
| 1,2-Propylenglykol | 7,00 |
| Sorbit, 70%-ig | 5,00 |
| 1,2-Propylenglykolmonomethylether | 17,00 |
| Natriumcyclamat | 0,30 |
| Allantoin | 0,25 |
| Azulen | 0,05 |
| Entsalztes Wasser | 59,30 |

Vor der Anwendung wird das Konzentrat im Verhältnis 1:10 mit Wasser verdünnt.

**Patentansprüche**

1. Verwendung von Hesperidin zur Herstellung von Zahn- und Mundpflegemitteln auf Basis der für diesen Zweck üblichen Zusatz- und Aufbaustoffe.

2. Verwendung von Hesperidin zur Herstellung von Zahn- und Mundpflegemitteln nach Anspruch 1, gekennzeichnet durch einen Zusatz von 0,05 bis 1,5 Gew.-% Hesperidin, bezogen auf die Gesamtzusammensetzung.

3. Verwendung von Hesperidin zur Herstellung von Zahn- und Mundpflegemitteln nach Anspruch 2, gekennzeichnet durch einen Zusatz von 0,1 bis 1,0 Gew.-% Hesperidin, bezogen auf die Gesamtzusammensetzung.

4. Zahn- und Mundpflegemittel mit einem Gehalt an Hesperidin, gekennzeichnet durch einen zusätzlichen Gehalt an Harnstoff.

5. Zahn- und Mundpflegemittel nach Anspruch 4, gekennzeichnet durch einen Gehalt an 5 bis 15 Gew.-% Harnstoff, berechnet auf die Gesamtzusammensetzung.

6. Zahn- und Mundpflegemittel nach Anspruch 4 oder 5, vorliegend in Form einer Zahnpasta auf wässriger Basis, enthalten Calciumcarbonat als alleiniges oder überwiegendes Poliermittel, 0 bis 15 Gew.-% Harnstoff, 0,5 bis 1 Gew.-%, bezogen auf die Gesamtzusammensetzung, eines Alkalisalzes einer $C_{12}$–$C_{22}$-Fettsäure, mit einem pH-Wert oberhalb 7,5, gekennzeichnet durch einen zusätzlichen Gehalt an Hesperidin.

**Claims**

1. Use of hesperidine for the manufacturing of compositions for dental and oral hygiene on the basis of the usual additives and excipients.

2. Use of hesperidine for the manufacturing of compositions for dental and oral hygiene according to claim 1, characterized by the addition of 0,05 to 1,5% by weight of hesperidine, calculated to the total composition.

3. Use of hesperidine for the manufacturing of compositions for dental and oral hygiene according to claim 2, characterized by the addition of 0,1 to 1,0% by weight of hesperidine, calculated to the total composition.

4. Composition for dental and oral hygiene containing hesperidine, characterized in that it contains an additional amount of urea.

5. Composition for dental and oral hygiene according to claim 4, characterized in that it contains 5 to 15% by weight of urea, calculated to the total composition.

6. Composition for dental and oral hygiene according to claim 4 or 5, being present as a toothpaste on an aqueous basis and containing calcium carbonate as sole or major polishing agent, 0 to 15% by weight of urea, 0,5 to 1% by weight, calculated to the total composition, of an alkali salt of a $C_{12}$–$C_{22}$-fatty acid and having a pH-value of more than 7,5, characterized in that it contains an additional amount of hesperidine.

**Revendications**

1. Utilisation d'hesperidine dans la fabrication des produits pour l'hygiène dentaire et buccale sur la base des additifs et des excipients usuels.

2. Utilisation d'hesperidine dans la fabrication des produits pour l'hygiène dentaire et buccale selon revendication 1, caractérisés par l'addition de 0,05 à 1,5% en poids d'hesperidine, calculé sur la composition totale.

3. Utilisation d'hesperidine dans la fabrication des produits pour l'hygiène dentaire et buccale selon revendication 2, caractérisé par l'addition de 0,1 à 1,0% en poids d'hesperidine, calculé sur la composition totale.

4. Produit pour l'hygiène dentaire et buccale contenant d'hesperidine, caractérisé par le fait qu'il renferme l'urée.

5. Produit pour l'hygiène dentaire et buccale selon revendication 4, caractérisé par le fait qu'il contient 5 à 15% en poids d'urée, calculé sur la composition totale.

6. Produit pour l'hygiène dentaire et buccale selon revendication 4 ou 5, présent sous forme de dentifrice sur base aqueuse et contenant carbonate de calcium comme agent abrasif unique ou majeur, 0 à 15% en poids d'urée, 0,5 à 1% en poids, calculé sur la composition totale, de sel alcalin d'un acide gras $C_{12}$–$C_{22}$ et ayant une valeur pH de plus de 7,5, caractérisé par le fait qu'il renferme l'hesperidine.